(19) Europäisches Patentamt
European Patent Office
Office européen des brevets

(11) Publication number: 0 370 795 A2

(12) **EUROPEAN PATENT APPLICATION**

(21) Application number: 89312136.8

(51) Int. Cl.5: C07D 203/02, C07D 203/08

(22) Date of filing: 22.11.89

(30) Priority: 25.11.88 JP 296022/88

(43) Date of publication of application: 30.05.90 Bulletin 90/22

(84) Designated Contracting States: BE CH DE ES FR GB IT LI NL SE

(71) Applicant: NIPPON SHOKUBAI KAGAKU KOGYO CO. LTD.
1-1, Koraibashi, 4-chome
Chuo-ku Osaka-shi Osaka-fu(JP)

(72) Inventor: Shimasaki, Yuuji
1-305, Nishimachi 5-ban
Takatsuki-shi Osaka-fu(JP)
Inventor: Tsuneki, Hideaki
5-5-4, Nakanobu Shinagawa-ku
Tokyo(JP)
Inventor: Hino, Youichi
1-80-1, Ohtorinishi-machi
Sakai-shi Osaka-fu(JP)
Inventor: Ueshima, Michio
13-63, Hoshoen
Takarazuka-shi Hyogo-ken(JP)

(74) Representative: Myerscough, Philip Boyd et al
J.A.Kemp & Co. 14, South Square Gray's Inn
London, WC1R 5EU(GB)

(54) **Method of producing aziridine compounds.**

(57) A method of producing an aziridine compound of the general formula

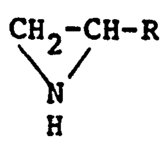

(I)

wherein R represents a hydrogen atom or a methyl or ethyl group,
which comprises subjecting an alkanolamine represented by the following formula

$\underset{X}{CH_2}-\underset{Y}{CH}-R$ (II)

wherein R is as defined, X represents the OH group or the $NH_2$ group, and Y represents the $NH_2$ group when X is the OH group, and the OH group when X is the $NH_2$ group,
to intramolecular dehydration reaction in the gaseous phase in the presence of a catalyst; wherein the catalyst contains acid sites having an acid-base strength (Ho) of from +4.0 to +7.0 and base sites having an acid-base strength of from +7.0 to +9.3.

EP 0 370 795 A2

## METHOD OF PRODUCING AZIRIDINE COMPOUNDS

An aziridine compound is a cyclic amine having an amino group with high reactivity. It is a very useful compound which has already been used widely in industry as a material for medicines, agricultural chemicals and also for amine-type polymers which are useful as textile treating agents.

A method comprising treating a sulfuric ester of an alkanolamine in the liquid phase with a concentrated alkali is generally well known for the production of aziridine compounds from alkanolamines. This method has already been commercialized as a method of producing ethylenimine. However, it has many defects when practiced commercially. For example, it has low productivity because large amounts of sulfuric acid and an aikali are used as subsidiary materials, and large amounts of inorganic salts having a low degree of utilization occur as by-products.

Some attempts to remove this defect of the liquid phase method by producing aziridine compounds directly by gaseous phase intramolecular dehydration reaction of alkanolamines in the presence of catalysts without using any subsidiary material have recently been reported. For example, Chem. Abs., 83, 163983 discloses a method involving the use of a tungsten oxide-type catalyst; U. S. Patent No. 4,301,036 discloses a method involving the use of a catalyst composed of tungsten oxide and silicon; and U. S. Patents Nos. 4289656, 4337175 and 4477591 disclose methods comprising using niobium- or tantalum-containing catalysts. However, all of these prior methods show a low conversion of the alkanolamines. Even when the conversion is relatively high, the proportion of by-products such as acetaldehyde or ethylamine formed is high, and therefore, the selectivity for the aziridine compounds is low.

Furthermore, with regard to the method of producing aziridine compounds by the gaseous phase intramolecular dehydration reaction of alkanolamines, European Laid-Open Patent Publication No. 227,461 dislcoses a silicon-containing catalyst, and European Laid-Open Patent Publications Nos. 228,898 and 230,776 disclose phosphorus-containing catalysts. The use of these catalysts have been found to solve the above problems. It is known however that when aziridine compounds are produced generally by intramolecular dehydration reaction of alkanolamines in the gaseous phase, the starting material is lost because various by-products such as carbonyl compounds or amine compounds corresponding to the starting alkanolamines form, and in particular, the carbonyl compounds react with the starting alkanolamines to form Schiff bases. Accordingly, with regard to the elimination of these defects, the above-mentioned catalysts have not proved to be entirely satisfactory.

It is an object of this invention therefore to provide a method of producing aziridine compounds in high yields and with high selectivity by the gaseous phase intramolecular dehydration reaction of alkanolamines while inhibiting the formation of by-products such as carbonyl compounds.

The present inventors extensively made investigations in order to achieve this object, and have now found that it is effective to perform the above reaction by using a catalyst characterized by having acid sites and base sites each with an acid-base strength (Ho) within a specific range.

Thus, according to this invention, there is provided a method of producing an aziridine compound of the general formula

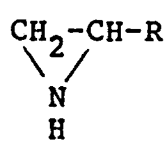 (I)

wherein R represents a hydrogen atom or a methyl or ethyl group,
which comprises subjecting an alkanolamine represented by the following formula

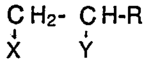 (II)

wherein R is as defined, X represents the OH group or the $NH_2$ group, and Y represents the $NH_2$ group when X is the OH group, and the OH group when X is the $NH_2$ group,
to intramolecular dehydration reaction in the gaseous phase in the presence of a catalyst; wherein the catalyst has acid sites having an acid-base strength (Ho) of from +4.0 to +7.0, preferably +4.8 to +7.0, and base sites having an acid-base strength of from +7.0 to +9.3.

Examples of the alkanolamine of general formula (II) used as a starting material in this invention include (a) monoethanolamine, (b) isopropanolamine, (c) 2-amino-1-propanol, (d) 1-amino-2-butanol, and (e) 2-amino-1-butanol. The aziridine compounds corrresponding to these monoalkanolamines of formula (I) are

respectively (a′) ethylenimine, (b′) 2-methyl-ethylenimine, (c′) 2-methyl-ethylenimine, and (d′) 2-ethyl-ethylenimine.

The catalysts that can be used in this invention may be any catalysts which contain acid sites and base sites having acid-base strength (Ho) within the specific ranges described above, and are not particularly limited as to their components or carriers. Suitable catalysts are those obtained by combining acid metal oxides with oxides of alkali metals and/or alkaline earth metals. Examples of the acid oxides are oxides of phosphorus, silicon, germanium, tin, lead, antimony, bismuth, group IIIA elements of the periodic table, transition metal elements of Groups I to VIII, lanthanide elements and actinide elements. On the other hand, examples of the oxides of alkali metals or alkaline earth metals include oxides of Na, K, Rb, Cs, Mg, Ca, Sr and Ba. They may be used in combination with each other. Specific examples are those shown in the above-cited prior art relating to the production of aziridine compounds by intramolecular dehydration of alkanolamines in the gaseous phase.

(1) Phosphorus-containing catalysts of the general formula

$X_aP_bM_cO_d$

wherein X represents at least one element selected from the group consisting of elements of Group IIIA of the periodic table, silicon, germanium, tin, lead, antimony, bismuth, transition metal elements, lanthanide elements and actinide elements (examples of these elements are B, Al, Tl, Si, Sn, Sb, Bi, Cu, Zn, Cd, Y, Ti, Zr, Nb, Ta, W, Mn, Fe, Ni, La, Ce, Eu and Th), P represents phosphorus, M represents at least one element selected from the group consisting of alkali metal elements and alkaline earth metal elements (examples of these elements are Li, Na, K, Rb, Cs, Sr and Ba), O represents oxygen, when a is 1, b is 0.01 to 6 and c is 0 to 3, and d is a number determined by the values of a, b and c and the states of bonding of the various constituent elements,

described in European Laid-Open Patent Publication No. 230,776.

(2) Phosphorus-containing catalysts of the general formula

$X_aP_bY_cO_d$

wherein X represents at least one element selected from the group consisting of alkali metal elements and alkaline earth metal elements, P represents phosphorus, Y represents at least one element selected from the group consisting of boron, aluminum, silicon, sulfur, titanium, copper, yttrium, zirconium, niobium, tuntalum, tungsten, lanthanum, cerium and thorium, O represents oxygen, when a is 1, b is 0.05 to 3 and c is 0 to 1, and d is a number determined by the values of a, b and c and the state of bonding of the various constituent elements,

disclosed in European Laid-Open Patent Publication No. 228,898.

(3) Silicon-containing catalysts of the general formula

$Si_aX_bY_cO_d$

wherein X represents at least one element selected from the group consisting of alkali metal elements and alkaline earth metal elements, Y represents at least one element selected from the group consisting of boron, aluminum, titanium, zirconium, tin, zinc and cerium, O represents oxygen, when a is 1, b is 0.005 to 1, and c is 0 to 1, and d is a number determined by the values of a, b and c, and the state of bonding of the various constituent elements,

disclosed in European Laid-Open Patent Publication No. 227,461.

There is no particular limitation on the method of preparing the catalyst used in this invention, and it may be produced by any methods generally practiced heretofore. Controlling of the step of preparing the catalyst should be performed carefully because the Ho value is affected not only by the amounts of the catalyst ingredients used but also by the catalyst calcination temperature and the amount of water present during catalyst preparation. It is important in this invention to use a catalyst containing acid sites having an Ho value of from +4.0 to +7.0, preferably from +4.8 to +7.0, and base sites having an Ho of +7.0 to +9.3. If the catalyst does not contain acid sites or the Ho of the base sites is higher than +9.3 (the base strength is strong), the catalyst activity is low, the formation of a carbonyl compound by deammoniation reaction increases, and the selectivity for the aziridine compound decreases. On the other hand, if the catalyst does not contain base sites or the Ho of the acid sites is lower than +4.0 (the acid strength is strong), the catalyst activity becomes high, but the formation of amine compounds or the formation of pyrazines by intermolecular condensation increases, and the selectivity for the aziridine compound decreases. It is important that in this type of reaction, acid sites and base sites existing on the surface of the catalyst should act synergistically. By using the catalyst containing acid sites and base sites with Ho in specific ranges in accordance with the method of this invention, such a synergistic action becomes possible for the first time, and the desired aziridine compound can be produced in high yields and with high selectivities while inhibiting the formation of by-product carbonyl compounds.

The determination of the presence or absence of acid sites and base sites and the measurement of the

acid-base strength function (Ho) can be easily performed by the Hammett's indicator method. Specifically, in the measurement of Ho of acid sites, several kinds of Hammett's indicators having Ho values smaller than +7.0 are provided, and individually added to a sample. The lowest Ho of the Hammett's indicators which caused color change is defined as the Ho of the acid sites of this sample. Likewise, in the measurement of Ho of base sites, several kinds of Hammett's indicators having Ho values larger than +7.0 are provided and individually added to the sample. The highest Ho of the Hammett's indicators which caused color change is defined as the Ho of the base sites of that sample.

The method of this invention is performed by passing the alkanolamine in the gaseous phase over the catalyst. The reaction conditions may be conventional ones which are, for example, described in the prior art. Generally, the reaction temperature is 100 to 500 °C, preferably 200 to 450 °C. The reaction pressure is atmospheric, reduced or elevated pressure. As required, alkanolamine may be diluted with an inert gas such as nitrogen or helium. Usually, the starting gas is fed onto the catalyst in an alkanolamine concentration of 1 to 100 % by volume. The space velocity, which may differ depending upon the catalyst, the reaction temperature, the reaction material, etc., is 100 to 30,000 $hr^{-1}$, preferably 500 to 10,000 $hr^{-1}$. The reactor may be of a fixed bed flowing type or a fluidized bed type.

The following examples illustrate the present invention specifically.

The definitions of the conversion of the alkanolamine, the selectivity for the aziridine compound and the selectivity for the carbonyl compound in the following examples are in accordance with the following.

$$\text{Conversion of the alkanolamine (mole \%)} = \frac{\text{Moles of the alkanolamine consumed}}{\text{Moles of the alkanolamine fed}} \times 100$$

$$\text{Selectivity for the aziridine compound (mole \%)} = \frac{\text{Moles of the aziridine compound formed}}{\text{Moles of the alkanolamine consumed}} \times 100$$

$$\text{Selectivity for the carbonyl compound (mole \%)} = \frac{\text{Moles of the carbonyl compound formed}}{\text{Moles of the alkanolamine consumed}} \times 100$$

## EXAMPLE 1

Barium hydroxide octahydrate (47.3 g) and 30 g of silicon oxide were suspended in 100 g of water, and with thorough stirring, the suspension was heated at 90 °C to concentrate it and give a white slurry-like mixture. The mixture was dried overnight at 120 °C in air, pulverized to a size of 3.5 mesh, and calcined at 500 °C for 2 hours to prepare a catalyst.

The catalyst was pulverized to a size of 100 mesh, and dried at 300 °C for 24 hours. A portion (about 0.1 g) of the dried catalyst was put in about 1 ml of absolute benzene to prepare a sample. Nine such samples in total were prepared, and to these samples, dicinnamalacetone (Ho=-3.0), p-dimethylaminoazobenzene (Ho= +3.3), phenylazonaphthylamine (Ho= +4.0), methyl red (Ho= +4.8), neutral red (Ho= +6.8), bromothymol blue (Ho= +7.2), m-nitrophenol (Ho= +8.3), phenolphthalein (Ho= +9.3) and 2,4,6-trinitroaniline (Ho= +12.2) were respectively added as 2 to 3 drops of their benzene solutions. They were individually stirred, and left to stand for 24 hours at room temperature. Then, by the method described above, the Ho values of the sample were measured. It was found that the Ho values of the acid sites and base sites of this catalyst were +6.8 and +9.3, respectively.

The catalyst (20 ml) was filled in a stainless steel reactor having an inside diameter of 16 mm, and then

the reaction tube was immersed in a molten salt bath at 400 °C. A starting gas composed of monoethanol amine and nitrogen in a volume ratio of 5:95 was passed through the reaction tube at a space velocity of 1,500 $hr^{-1}$ (STP), and reacted. The reaction product was analyzed by gas chromatography, and the results shown in Table 1 were obtained.

EXAMPLE 2

Example 1 was repeated except that 2-amino-1-butanol was used in the starting gas instead of monoethanolamine. The results are shown in Table 1.

EXAMPLE 3

A catalyst was prepared from 30 g of silicon oxide, 0.56 g of potassium hydroxide and 1.6 g of titanium oxide as materials as in Example 1. Its Ho values were measured as in Example 1, and the gaseous phase intramolecular dehydration reaction of monoethanolamine was carried out as in Example 1 using the catalyst so prepared. The results are shown in Table 1.

EXAMPLE 4

Example 1 was repeated except that isopropanolamine was used instead of monoethanolamine in the starting gas, and the catalyst prepared in Example 3 was used instead of the catalyst used in Example 1. The results are shown in Table 1.

EXAMPLE 5

Zinc oxide (48.8 g) was suspended in 100 ml of water, and 55.4 g of 85 % by weight ortho-phosphoric acid was added. With sufficient stirring, the mixture was evaporated to dryness on a hot water bath. The solid product was dried overnight at 120 °C in air, pulverized to a size of 9 to 5 mesh, and calcined at 550 °C for 2 hours to prepare a catalyst.

The Ho values of the catalyst were measured as in Example 1, and monoethanolamine was reacted as in Example 1 except that the catalyst so prepared was used instead of the catalyst used in Example 1.

EXAMPLE 6

Example 1 was repeated except that the catalyst prepared in Example 5 was used instead of the catalyst used in Example 1, and isopropanolamine was used instead of monoethanolamine in the starting gas. The results are shown in Table 1.

EXAMPLE 7

A solution of 40.2 g of triammonium phosphate in 300 ml of water was added to a solution of 130.3 g of lanthanum nitrate hexahydrate in 300 ml of water with stirring. The resulting precipitate was collected by filtration, washed with water, dried overnight at 120 °C in air, then pulverized to a size of 9 to 5 mesh, and calcined at 750 °C for 2 hours to prepare a catalyst. The Ho values of the catalyst were measured as in Example 1.

Monoethanolamine was reacted in the same way as in Example 1 except that the catalyst so prepared

was used instead of the catalyst used in Example 1.

## EXAMPLE 8

Example 1 was repeated except that the catalyst prepared in Example 7 was used instead of the catalyst used in Example 1, and 2-amino-1-butanol was used instead of monoethanolamine in the starting gas. The results are shown in Table 1.

## EXAMPLE 9

A solution of 40.2 g of triammonium phosphate in 300 ml of water was added to a solution of 112.5 g of aluminum nitrate nonahydrate in 300 ml of water with stirring. The resulting precipitate was collected by filtration, and washed with water. A solution of 28.4 g of barium hydroxide octahydrate in 100 ml was added, and these materials were well kneaded. The kneaded mixture was dried overnight at 120 °C for 2 hours, pulverized to a size of 9 to 5 mesh, and calcined at 900 °C for 2 hours to prepare a catalyst. The Ho values of the catalyst were measured.

Example 1 was repeated except that the catalyst so prepared was used instead of the catalyst used in Example 1. The results are shown in Table 1.

## EXAMPLE 10

Example 1 was repeated except that the catalyst prepared in Example 9 was used instead of the catalyst used in Example 1, and isopropanolamine was used instead of monoethanolamine in the starting gas. The results are shown in Table 1.

## EXAMPLE 11

Cerous oxide (49.2 g) was suspended in 100 ml of water, and 34.6 g of 85 % by weight orthophosphoric acid was added. With sufficient stirring, the mixture was heated and concentrated, and evaporated to dryness on a hot water bath. The solid product was dried overnight at 120 °C in air, pulverized to a size of 9 to 5 mesh, and calcined at 600 °C for 2 hours. The Ho values of the catalyst were measured as in Example 1.

Example 1 was repeated except that the catalyst so prepared was used instead of the catalyst used in Example 1. The results are shown in Table 1.

## EXAMPLE 12

A solution of 118.1 g of calcium nitrate tetrahydrate in 200 ml of water was heated to 80 °C, and with stirring, a solution of 46.2 g of diammonium phosphate in 100 ml of water was added. Then, aqueous ammonia was added to maintain the solution basic, and the solution was aged for 30 minutes, cooled, filtered, and then washed with water to give a white solid. The solid was dried at 120 °C, calcined at 500 °C for 2 hours, and pulverized to a size of 3.5 mesh to prepare a catalyst. The Ho values of the catalyst were measured as in Example 1.

Example 1 was repeated except that the catalyst prepared above was used instead of the catalyst used in Example 1. The results are shown in Table 1.

## EXAMPLE 13

Example 1 was repeated except that the catalyst prepared in Example 12 was used instead of the catalyst used in Example 1, and isopropanolamine was used instead of monoethanolamine in the starting gas. The results are shown in Table 1.

## EXAMPLE 14

A catalyst was prepared as in Example 12 except that 63.1 g of barium hydroxide octahydrate and 49.4 g of magnesium phosphate 22-hydrate were used instead of calcium nitrate and diammonium phosphate in the preparation of the catalyst in Example 12.

The Ho values of the catalyst were measured as in Example 1. Monoethanolamine was reacted as in Example 1 except that the catalyst so prepared was used instead of the catalyst used in Example 1. The results are shown in Table 1.

## EXAMPLE 15

Example 1 was repeated except that the catalyst prepared in Example 14 was used instead of the catalyst used in Example 1, and 2-amino-1-butanol was used instead of monoethanolamine in the starting gas. The results are shown in Table 1.

## EXAMPLE 16

Silicon oxide (24 g) and 0.174 g of boron oxide, as powders, were well mixed in a mortar. The mixture was kneaded with 4.25 g of sodium nitrate and 1.73 g of 85 % by weight phosphoric acid while adjusting moisture with water. The resulting wet solid was dried, calcined and pulverized as in Example 1 to prepare a catalyst. The Ho values of the catalyst were measured as in Example 1.

Monoethanolamine was reacted as in Example 1 except that the catalyst so prepared was used instead of the catalyst used in Example 1.

## EXAMPLE 17

Example 1 was repeated except that the catalyst prepared in Example 16 was used instead of the catalyst used in Example 1, and isopropanolamine was used instead of monoethanolamine in the starting gas. The results are shown in Table 1.

## EXAMPLE 18

Cesium carbonate (11.40 g), 11.9 g of ammonium phosphate, 1.74 g of magnesium hydroxide and 25.5 g of aluminum oxide were concentrated in 200 ml of water to give a white slurry-like substance. It was dried overnight at 120 °C in air, pulverized to a size of 3.5 mesh, and calcined at 700 °C for 3 hours to give a catalyst. Its Ho values were measured as in Example 1.

Monoethanolamine was reacted in the same way as in Example 1 except that the catalyst so prepared was used instead of the catalyst used in Example 1. The results are shown in Table 1.

## COMPARATIVE EXAMPLE 1

A catalyst was prepared as in Example 1 by using magnesium oxide alone as a material. The Ho values of this catalyst were measured as in Example 1. Since it did not discolor neutral red (Ho = +6.8), it was determined that the catalyst has no acid site. On the other hand, since it discolored 2,4,6-trinitroaniline (Ho = +12.2), it was determined that this catalyst had base sites having an Ho value of +12.2.

Monoethanolamine was reacted as in Example 1 except that the catalyst so prepared was used instead of the catalyst used in Example 1. The results are shown in Table 1.

## COMPARATIVE EXAMPLE 2

A catalyst was prepared as in Example 1 except that 30 g of silicon dioxide and 5.1 g of aluminum oxide were used as materials. The Ho values of the catalyst were measured in the same way as in Example 1. Since it discolored dicinnamalacetone (Ho = -3.0), it was determined that the catalyst has acid sites having an Ho value of -3.0. Furthermore, since it did not discolor bromothymol blue (Ho = +7.2), it was determined that the catalyst has no base site.

Monoethanolamine was reacted in the same way as in Example 1 except that the catalyst so prepared was used instead of the catalyst used in Example 1.

## COMPARATIVE EXAMPLE 3

A catalyst was prepared as in Example 1 except that silicon oxide alone was used as a material. Since it discolored phenylazonaphthylamine (Ho = +4.0), it was determined that it has an acid site having an Ho value of +4.0. Furthermore, since it did not discolor bromothymol blue (Ho = +7.2), it was determined that the catalyst has no base site.

Monoethanolamine was reacted as in Example 1 except that the catalyst so prepared was used instead of the catalyst used in Example 1. The results are shown in Table 1.

Table 1

| Run | Catalyst | | | Reaction conditions and results | | | | |
|---|---|---|---|---|---|---|---|---|
| | Catalyst composition (atomic ratio excepting oxygen) | Acid-base strength (Ho) | | Reaction temperature (°C) | Starting alkanolamine | | Selectivity for the aziridine compound (mole %) | Selectivity for the carbonyl compound (mole %) |
| | | Acid sites | Base sites | | Substituent R | Conversion (mole %) | | |
| Example 1 | $Si_1Ba_{0.3}$ | 6.8 | 9.3 | 400 | H | 53.4 | 72.8 | 9.2 |
| Example 2 | | | | 400 | $C_2H_5$ | 54.0 | 78.0 | 8.5 |
| Example 3 | $Si_1K_{0.02}Ti_{0.04}$ | 4.8 | 8.3 | 380 | H | 47.2 | 83.9 | 7.8 |
| Example 4 | | | | 380 | $CH_3$ | 48.5 | 86.1 | 6.9 |
| Example 5 | $Zn_1P_{0.8}$ | 4.8 | 8.3 | 410 | H | 44.7 | 69.0 | 13.2 |
| Example 6 | | | | 410 | $CH_3$ | 49.2 | 67.8 | 14.1 |
| Example 7 | $La_1P_{0.9}$ | 4.0 | 8.3 | 380 | H | 52.9 | 63.4 | 12.5 |
| Example 8 | | | | 380 | $C_2H_5$ | 55.1 | 64.7 | 13.0 |
| Example 9 | $Al_1Ba_{0.3}P_{0.9}$ | 4.8 | 9.3 | 410 | H | 74.9 | 76.6 | 9.4 |
| Example 10 | | | | 410 | $CH_3$ | 76.9 | 78.1 | 8.9 |

- to be continued -

Table 1 (continued)

| Run | Catalyst | | | Reaction conditions and results | | | | |
|---|---|---|---|---|---|---|---|---|
| | Catalyst composition (atomic ratio excepting oxygen) | Acid-base strength (Ho) | | Reaction temperature (°C) | Starting alkanolamine | | Selectivity for the aziridine compound (mole %) | Selectivity for the carbonyl compound (mole %) |
| | | Acid sites | Base sites | | Substituent R | Conversion (mole %) | | |
| Example 11 | $Ce_1Na_{0.6}P_1$ | 6.8 | 8.3 | 410 | H | 71.0 | 75.4 | 13.2 |
| Example 12 | $Ca_1P_{0.7}$ | 6.8 | 9.3 | 360 | H | 62.9 | 80.4 | 9.7 |
| Example 13 | | | | 350 | $CH_3$ | 61.9 | 78.1 | 9.5 |
| Example 14 | $Mg_{0.7}Ba_{0.6}P_{0.5}$ | 6.8 | 8.3 | 350 | H | 58.9 | 81.8 | 7.9 |
| Example 15 | | | | 340 | $C_2H_5$ | 58.3 | 82.0 | 7.2 |
| Example 16 | $Na_1P_{0.3}Si_8B_{0.1}$ | 4.0 | 8.3 | 390 | H | 68.3 | 76.3 | 8.1 |
| Example 17 | | | | 380 | $CH_3$ | 67.1 | 77.6 | 8.0 |
| Example 18 | $Mg_{0.3}Cs_{0.7}P_{0.9}Al_{5.0}$ | 4.8 | 8.3 | 400 | H | 72.5 | 81.1 | 6.1 |
| Comp. Example 1 | Mg | - | 12.2 | 420 | H | 17.7 | 0 | 73.6 |
| Comp. Example 2 | $Si_1Al_{0.2}$ | -3.0 | - | 350 | H | 91.8 | 8.3 | 26.5 |
| Comp. Example 3 | Si | 4.0 | - | 420 | H | 11.4 | 53.2 | 17.1 |

## Claims

1. A method of producing an aziridine compound of the formula

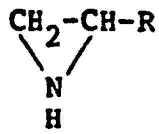 (I)

wherein R represents hydrogen, methyl or ethyl, which comprises subjecting an alkanolamine of the formula

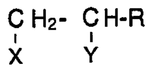 (II)

wherein R is as defined, X represents OH or $NH_2$, and Y represents $NH_2$ when X is OH, and OH when X is $NH_2$,
to an intramolecular dehydration reaction in the gaseous phase in the presence of a catalyst, characterised in that the catalyst contains acid sites having an acid-base strength (Ho) of from +4.0 to +7.0 and base sites having an acid-base strength of from +7.0 to +9.3.

2. A method according to claim 1 in which the catalyst comprises a combination of an acidic oxide and an oxide of an alkali metal and/or an alkaline earth metal.